(19) **Europäisches Patentamt**
**European Patent Office**
**Office européen des brevets**

(11) **EP 3 449 991 A1**

(12) **EUROPEAN PATENT APPLICATION**
published in accordance with Art. 153(4) EPC

(43) Date of publication:
**06.03.2019 Bulletin 2019/10**

(21) Application number: **17790000.8**

(22) Date of filing: **25.04.2017**

(51) Int Cl.:
**B01D 3/34** *(2006.01)*          **C10G 7/00** *(2006.01)*

(86) International application number:
**PCT/RU2017/000262**

(87) International publication number:
**WO 2017/188857 (02.11.2017 Gazette 2017/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **26.04.2016 RU 2016116633**

(71) Applicant: **Distilium Limited Liability Company
(LLC "DISTILIUM")**
**Moscow 143026 (RU)**

(72) Inventor: **Khamidullin, Rafik Nailovich
g. Kazan, 420140 (RU)**

(74) Representative: **Chimini, Francesco
Jacobacci & Partners S.p.A.
Piazza della Vittoria 11
25122 Brescia (IT)**

(54) **METHOD FOR REFINING LIQUIDS IN AN INERT GAS ENVIRONMENT**

(57)    The invention relates to the methods for carrying out heat and mass transfer processes for the liquid-liquid, gas-liquid, liquid-solid phase systems, including the processes of distillation, rectification, evaporation, degassing and other separation processes of liquid mixtures and suspensions. The invention can be used in the processes of rectification of thermally unstable products, the distillation of heavy oil residues, disposal of oil sludge, production of non- oxidized bitumen, distillation of glycerin, the ethanolamines, glycol, separation of solvents from extracts and products of refining, evaporation of solutions, degassing of gaz- liquid mixtures and many other processes that are implemented at a low pressure or high temperature The method for distilling liquids in an inert gas environment comprising introducing an initial mixture, withdrawing the separation products, introducing a non-condensable inert gas, heating the separation products, converting of one or more components of the initial mixture into a gas phase, separating one or more components of gas phase, and the ratio of the mass of the introduced inert gas to the mass of inert gas providing, in aggregate with the vapors of the substance to be removed the same volume of vapor phase at the point of its introduction, as in the process without the introduced inert gas at a lower pressure at the same process temperature, is 0,5-5,0. The inert gas is returned to interact with the initial mixture. The introduction and (or) withdrawal of the inert gas is carried out in several stages during the separation of the initial mixture. The highly volatile component of the initial mixture is released from the inert gas by cooling. The technical result: the development of a method for distilling mixtures without the use of low pressure and high temperature, reducing energy costs, reducing the environmental load.

FIG.2

**Description**

The invention is intended for carrying out heat and mass transfer processes for the liquid-liquid, gas-liquid, liquid-solid phase systems, including the processes of distillation, rectification, evaporation, degassing and other separation processes of liquid mixtures and suspensions. The invention can be used in the processes of rectification of thermally unstable products, the distillation of heavy oil residues, disposal of oil sludge, production of non- oxidized bitumen, distillation of glycerin, the ethanolamines, glycol, separation of solvents from extracts and products of refining, evaporation of solutions, degassing of gaz- liquid mixtures and many other processes that are implemented at a low pressure or high temperature. The field of application of this invention is petrochemical, chemical, pharmaceutical, food, metallurgical and other industries.

Known a method of producing non-oxidized bitumen from high resin oil using superheated steam, comprising heating the source oil up to 300°C, atmospheric distilling off distillates and obtaining the target product from the cube of the column.Moreover, the heated initial oil is supplied from the top of the column to the packed contact elements, and superheated steam with a temperature of 480-540°C is fed to the bottom of the column at a steam:oil ratio of (0,8-1,2):1 respectively (Patent RF № 2566775 from 27.10.2015, Farrakhov, M. I., Kirichenko S. M., Fahrutdinov R. Z., etc., OOO "ITC "Inzhekhim").

The disadvantages of this method are the high energy consumption for the production of steam, the need to utilize the products of condensation of water vapor with admixtures of the partial mixture.

The closest to the proposed method (prototype) is a method of stabilizing oil, including the supply of gas containing low-boiling hydrocarbons to the separator with the release of gas from oil enriched with low-boiling hydrocarbons, to obtain a stable oil. In order to increase the yield of the target product, a gas with a lower content of low-boiling hydrocarbons than in the gas released from oil is used to deliver, the gas is supplied to the gas space, and at the same time the gas supplied to the gas space of the separator is fed into the oil by bubbling (No. 1544790 23.02.90№7, Groshev, B., Bronstein, I. S., Kashtanov, and others, the national research Institute for the collection, preparation and transportation of oil and petroleum products).

The disadvantage of the prototype is the need to use low pressure.

The object of the present invention is to develop a method of separation of mixtures without the use of low pressure and high temperature, reducing energy costs, reducing the environmental load.

The task is solved by the fact that during the distillation of liquids in an inert gas environment, comprising introducing an initial mixture, withdrawing the separation products, introducing a non-condensable inert gas, heating the separation products, converting of one or more components of the initial mixture into a gas phase, separating one or more components of gas phase, **wherein** the ratio of the mass of the introduced inert gas to the mass of inert gas providing, in aggregate with the vapors of the substance to be removed the same volume of vapor phase at the point of its introduction, as in the process without the introduced inert gas at a lower pressure at the same process temperature, is 0,5-5,0. The inert gas is returned to interact with the initial mixture. The introduction and (or) withdrawal of the inert gas is carried out in several stages during the separation of the initial mixture. The highly volatile component of the initial mixture is released from the inert gas by cooling.

In the case of a single separation of the initial mixture (distillation), the method is implemented as follows.

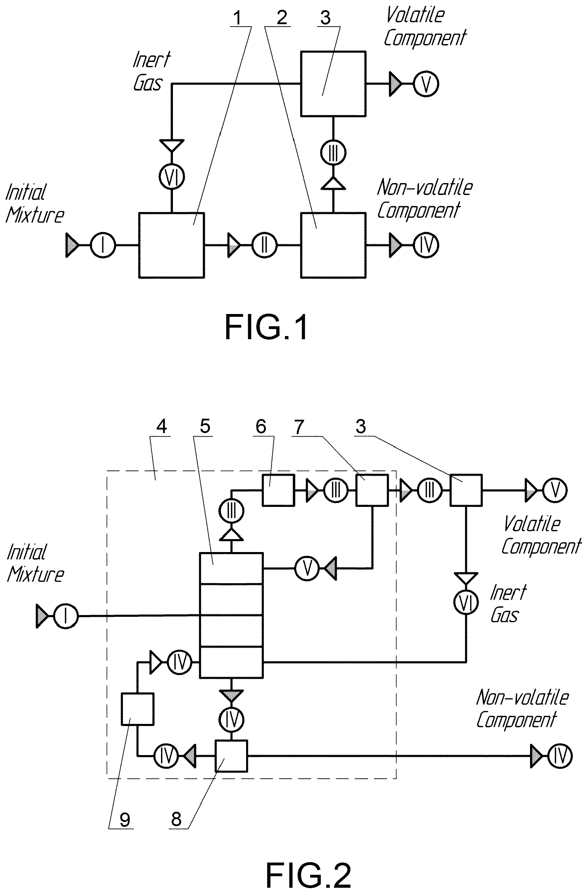

The separated components are fed into the mixer 1 (see Fig.1). Inert non-condensable gas, such as nitrogen, is also supplied here. The products are mixed, the released component due to the non-equilibrium state in the inert gas environment passes into the gas phase. The gas phase, comprising an inert gas and a vaporous volatile component, is separated from the liquid non-volatile component in the separator 2. The non-volatile component is directed to its destination. The inert gas is separated in the divider 3 from the volatile component, for example by cooling, and again sent to the mixer 1. The selected volatile component is sent to its destination.

Scheme, Fig. 1, consists of: 1 mixer, 2 separator, 3 divider. Flows: I, initial mixture, II mixture of inert gas and initial mixture, III mixture of volatile component and inert gas, IV working-age (non-volatile) component (group of components), V light-volatile component (group of components), VI inert non-condensable gas.

In the case of multiple separation of the initial mixture (rectification), the method is implemented as follows.

The initial mixture is fed to the separation into the rectification unit 4 (see Fig.2), where it is divided into volatile and non-volatile parts by multiple interaction of non-equilibrium gas and liquid flows.

Rectification unit 4 works as follows. The initial liquid mixture is fed to column 5 on a feeding plate located between the upper and lower plates. Inert gas is supplied to the lower part of the distillation column 5. Inside the column, the vapor phase is directed upwards towards the liquid flow of the initial mixture. In the process of liquid and gas interaction, the liquid phase is saturated with the non-volatile part of the initial mixture, the gas phase is saturated with the volatile one. In the lower part of the column after separator of non-volatile liquid component 8, a part of the liquid flow consisting of the non-volatile component is sent to the preheater 9 and then returns to the column, and the remaining part as the finished product is sent to its destination. At the top of the column, the gas flow consisting of a volatile component and an inert non-condensable gas is sent to the reflux condenser 6 to cool the flow. Further in the phlegm divider 7 it is divided into a liquid phlegm, which is sent back to the column, and the remaining part of the mixture of inert gas and a volatile component, that exits the rectification unit.

At the outlet of the rectification unit in the upper part, the remaining part of the mixture in the divider 3 is divided into a volatile component and an inert non-condensable gas, for example, by cooling. The inert gas returns to the interaction in the lower part of the column 5, and the volatile component is sent to its destination as the finished product.

Figure 2 consists of: 3 divider, 4 the rectifying unit 5 the distillation column, 6 dephlegmator, 7 phlegm divider, 8 divider the bottom of the fluid 9 heater. Flows: I initial mixture, III mixture of volatile component and inert gas, IV non-volatile component (group of components), V volatile component (group of components), VI inert non-condensable gas.

The molar (volume) fraction of any component of a mixture of ideal gases according to the equations of Clapeyron and Dalton is equal to the ratio of the partial pressure of the gas mixture component to the total pressure of the mixture:

$$y_a = \frac{p_a}{P}$$

where: $y_a$ is the volume fraction of component A in the gas phase,
$p_a$ -is the partial pressure of component A in the gas phase, Pa,
P is the system pressure, Pa.

On the other hand, the volume fraction of the component is determined by the ratio of the actual volume of the substance to the total volume of the system:

$$y_a = \frac{V_a}{V}$$

where: $y_a$ -volume content of component A in the gas phase,
$V_a$ - volume occupied by component a, $m^3$,
V-system volume, $m^3$.

The volume of component A is defined as the ratio of the mass of a substance to its density:

$$V_a = \frac{M_a}{\rho_a}$$

where: $V_a$ - volume occupied by component A, $m^3$,
$M_a$ - mass of substance A, kg.
$\rho_a$ - density of substance A, $kg/m^3$.

The Dalton equation can be represented in a modified form:

$$\frac{M_a}{V \cdot \rho_a} = \frac{p_a}{P}$$

where: $M_a$ - mass of substance A, kg,
V-system volume, $m^3$,
$\rho_a$ - density of substance A, $kg/m^3$,
$p_a$ -partial pressure of component A in the gas phase, Pa,
P -is the system pressure, Pa.

Transforming this equation, we obtain:

$$M_a = \frac{V \cdot p_a \cdot \rho_a}{P}$$

where: $M_a$ - mass of substance A, kg,
V-system volume, $m^3$,
$\rho_a$ - density of substance A, $kg/m^3$,
$p_a$ -partial pressure of component A in the gas phase, Pa,
P is the system pressure, Pa.

The density of gases is determined by the equation:

$$\rho_a = \frac{M_A \cdot P \cdot T_0}{22{,}4 \cdot P_0 \cdot T}$$

where: $M_A$ is the molar mass of the compound A, kg/kmol,
P-system pressure, Pa,
$T_0$ -temperature under normal conditions, °κ.
$P_0$ is the system pressure at normal conditions Pa,
T is the system temperature, °κ.

Calculating the mass ratio of the substance in the gas at different pressures we obtain:

$$\frac{M_{a1}}{M_{a2}} = \frac{\frac{V_1 \cdot p_a}{P_1} \cdot \frac{M_A \cdot P_1 \cdot T_0}{22{,}4 \cdot P_0 \cdot T_1}}{\frac{V_2 \cdot p_a}{P_2} \cdot \frac{M_A \cdot P_2 \cdot T_0}{22{,}4 \cdot P_0 \cdot T_2}}$$

or:

$$\frac{M_{a1}}{M_{a2}} = \frac{V_1 \cdot p_a \cdot M_A \cdot P_1 \cdot T_0 \cdot P_2 \cdot 22{,}4 \cdot P_0 \cdot T_2}{V_2 \cdot p_a \cdot M_A \cdot P_2 \cdot T_0 \cdot P_1 \cdot 22{,}4 \cdot P_0 \cdot T_1}$$

Excluding from the formula the values that do not change at different pressures, we obtain:

$$\frac{M_{a1}}{M_{a2}} = \frac{V_1 \cdot P_1 \cdot P_2 \cdot T_2}{V_2 \cdot P_2 \cdot P_1 \cdot T_1}$$

Provided that the volume of the system does not change and the temperature is constant, the change in the mass of the component A in the gas phase at different pressures of the system during the separation of liquid mixtures does not occur, or:

$$\frac{M_{a1}}{M_{a2}} = 1$$

Thus, when adding inert gas to a fixed volume of the system, where the process of separating the liquid mixtures takes place, the mass of vapor components in the gas phase will be constant at a constant temperature and with a change in the absolute pressure of the system, adjusted for the deviations of the properties of the resulting gas phase from the properties of ideal gases, of the residual content of the components of the initial mixture in the inert gas and of the completeness of the output of the components to the gas phase. The value of the compressibility factor for gases, taking into account the deviation of the real gases from the ideal may be in the range of 0,2-20 units.

For operating pressures and temperatures ranges of the scope of the present invention, taking into account the above factors, the ratio of the mass of the introduced inert gas to the mass of inert gas providing, in aggregate with the vapors of the substance to be removed the same volume of vapor phase at the point of its introduction, as in the process without the introduced inert gas at a lower pressure at the same process temperature, is 0,5-5,0. As an inert gas, use any chemically non-interacting gases that do not change their state of aggregation during interaction with the components of the mixture to be separated. For example, nitrogen, in the process of separation of heavy residues of oil, carbon dioxide, methane, etc.

In the process of separation of mixtures by multiple interaction of non-equilibrium flows, the inert gas, leaving from the upper part of the column will have mainly volatile component in its composition. It is technically impossible to completely separate the inert gas from the impurities of the initial mixture. When adding an inert gas to the bottom of the column, a part of the volatile component will dilute the waste liquid with the predominant content of the non-volatile component. In order to minimize the transfer of components from the top of the column to the bottom, the input and output of inert gas is carried out in several stages. For example, an inert gas is introduced into the lower part, and is removed from the middle part, another inert gas circuit is introduced in the middle part, and displayed in the upper one.

In addition, transporting inert gas through the entire column can increase the total pressure drop across the column. The pressure drop across the entire column can be reduced with a step-by-step inert gas inlet-outlet.

In some processes, conditions can be created under which it will be economically advantageous to remove some of the inert gas on the overlying plate from the column, for example to reduce the high hydraulic resistance of the column section. Or vice versa, to introduce additional inert gas on any of the plates, for example, to provide the necessary hydrodynamic mode of operation.

The separation of the inert gas and the components of the initial mixture by cooling is the least expensive process.

Implementation of the claimed method of separating a liquid mixture in an inert gas medium is explained by the schemes shown in Fig. 1 and 2.

**Example 1.** The allocation of propylene carbonate from petroleum extract (see Fig.1, table. 1, 2). A vacuum of 251,2 Pa is required to isolate the propylene carbonate to the residual content in the initial mixture of 0,05 %,. The actual volumetric flow rate of the vapor phase is 772,6 $m^3$/h per 105 kg of the initial mixture. To ensure a similar flow rate of the separated mixture vapor phase (772,6 $m^3$/h) at an absolute pressure of 100 kPa, 560,5 kg/h of Nitrogen must be added to the mixture. With a nitrogen density of 1,15 kg/$m^3$ (20 °C), its volume will be 487,4 $m^3$.

The composition of the initial mixture, the separation products without the addition of nitrogen at an absolute pressure of 251,2 Pa and with the addition of nitrogen at a pressure of 100 kPa are presented in table 1. The flow rate in the inert gas environment and without it are presented in table 2.

Table 1. Composition of initial mixture and residue of separation products:

| № | Component | Composition of the component, (mass) % | | | | |
|---|---|---|---|---|---|---|
| | | Initial mixture | $P_{abs.}$ = 0,2512 kPa, nitrogen free | | $P_{abs.}$ = 100,0 kPa, with nitrogen | |
| | | | Gas phase after separation | The residue after separation | Gas phase after separation | The residue after separation |
| 1 | fraction $t_к$= 365 | 0,38 | 2,81 | 0,24 | 0,03 | 0,24 |
| 2 | fraction $t_к$=383 | 0,46 | 2,13 | 0,36 | 0,02 | 0,36 |
| 3 | fraction $t_к$=401 | 0,83 | 2,12 | 0,76 | 0,02 | 0,76 |
| 4 | fraction $t_к$=419 | 1,31 | 1,74 | 1,29 | 0,02 | 1,29 |
| 5 | fraction $t_к$=441 | 2,86 | 1,52 | 2,93 | 0,02 | 2,93 |
| 6 | fraction $t_к$=470 | 6,82 | 0,95 | 7,16 | 0,01 | 7,16 |
| 7 | fraction $t_к$=497 | 15,35 | 0,57 | 16,19 | 0,01 | 16,19 |
| 8 | fraction $t_к$=524 | 17,17 | 0,15 | 18,14 | 0 | 18,14 |
| 9 | fraction $t_к$=551 | 15,31 | 0,03 | 16,17 | 0 | 16,17 |
| 10 | fraction $t_к$=576 | 19,24 | 0,01 | 20,33 | 0 | 20,33 |

(continued)

| No | Component | Composition of the component, (mass) % | | | | |
|---|---|---|---|---|---|---|
| | | Initial mixture | $P_{abs.}$ = 0,2512 kPa, nitrogen free | | $P_{abs.}$ = 100,0 kPa, with nitrogen | |
| | | | Gas phase after separation | The residue after separation | Gas phase after separation | The residue after separation |
| 11 | fraction $t_к$=606 | 9,27 | 0 | 9,79 | 0 | 9,79 |
| 12 | fraction $t_к$=631 | 4,41 | 0 | 4,65 | 0 | 4,65 |
| 13 | fraction $t_к$=660 | 1,84 | 0 | 1,94 | 0 | 1,94 |
| 14 | propylene carbonate | 4,76 | 87,98 | 0,05 | 0,87 | 0,05 |
| 15 | nitrogen | - | - | - | 99,0 | - |
| | total: | 100 | 100 | 100 | 100 | 100 |

Table 2. Composition of initial mixture and residue of separation products:

| No | | Flow rate, kg/h (m³/h) | |
|---|---|---|---|
| | | Separation of the mixture at an absolute pressure of 0,2512 kPa, | Separation of the mixture at an absolute pressure of 100 kPa in the inert gas environment(nitrogen) |
| 1 | Initial mixture: (t=20 °C), including nitrogen: | 105 (0,1065) | 665,5 (487,6) 560,5 (487,4) |
| 2 | Separation residue: (t=190 °C) | 99,37 (0,1175) | 99,37 (0,1175) |
| 3 | Gas phase after separation: (t=190 °C), including nitrogen: | 5,626 (772,6) | 566,1 (772,6) |

**Example 2.** Rectification of the styrene-ethylbenzene mixture (see Fig.2). To separate the styrene-ethylbenzene mixture, vacuum (absolute pressure from 4,29 to 16,18 kPa) and process temperatures from 67,1 to 86,89 °C are used to exclude the polymerization of styrene. The actual volume flow rate of the vapor phase in the cube is 155900 m³/h at 31000 kg /h of the initial mixture. To ensure a similar flow rate of the vapor phase (155900 m³/h) at the same temperature (86,89 °C) in the column cube at an absolute pressure of 98,7 kPa, 119422 kg/h of Nitrogen must be added to the mixture.

The characteristics of the process of rectification of styrene-ethylbenzene in two versions are presented in table 3.

Table 3. Rectification of a mixture of styrene-ethylbenzene

| No | Parameter name | Separation of the mixture under vacuum | Separation of the mixture in the inert gas environment (nitrogen) |
|---|---|---|---|
| 1 | Initial mixture, kg/h, (mass composition % - ethylbenzene 44,76, styrene 54,17, other 1,07) | 31000 | 31000 |
| 2 | nitrogen (fed to the cube of the column), kg/h | - | 119422 |
| 3 | Ethylbenzene yield, kg/h (content in liquid phase 95,8% Mass.) | 14368,4 | 133784,5 (including nitrogen 119418,7) |

(continued)

| № | Parameter name | Separation of the mixture under vacuum | Separation of the mixture in the inert gas environment (nitrogen) |
|---|---|---|---|
| 4 | Styrene yield (content in liquid phase 98,8% Mass.) | 16631,6 | 16637,5 (including nitrogen 3,3 кГ) |
| Top part of the column (top tray), | | | |
| 5 | Absolute pressure, kPa | 4,29 | 78,45 |
| 6 | Vapor consumption at the top of the column, m$^3$/h (kg/h) | 519400 (89060) | 184200 (203600) |
| | Bottom of the column (cube) | | |
| 7 | Absolute pressure, kPa | 16,18 | 98,07 |
| 8 | Vapor consumption at the bottom of the column, m$^3$/h (kg/h) | 155900 (88530) | 155900 (208800) |

The advantages of this invention are :

- the absence of the need to use high vacuum with similar separation efficiency indicators, reducing the temperature of the separation process, reducing energy consumption, reducing the environmental load by eliminating the formation of condensation products of water vapor, the absence of gas emissions from the components to be removed due to the return on the interaction.
- no need to reduce the pressure during the degassing of various liquid mixtures, such as crude oil, due to the introduction of an inert gas and when carrying out the process at high pressure.
- reduction of the dimensions of column devices due to the smaller relative pressure difference in the lower and upper parts of the column. Thus, at low absolute pressure of the process, the ratio of absolute pressure values in the lower and upper parts of the column is 3,653 (Example No. 2), and when rectifying in an inert gas environment, the ratio is 1,25, respectively, the volume flow rate of the vapor phase changes in the same number of times and as a consequence, the dimensions of the equipment change.
- simplification of the process of modernization of the existing production of separation of liquid mixtures, previously carried out under vacuum, by preserving the existing basic equipment design.
- increasing the efficiency of mass transfer devices due to the possibility of turbulence in flows using inert gas in the contact devices of column apparatus, mixers and separation devices.

The proposed technical solution is named by the applicant the process of "Distillation of liquids in an inert gas environment".

**Claims**

1. A method for distillation of liquids at inert gas environment, said method consisting of the steps of:

    introducing an initial mixture,
    withdrawing the separation products,
    introducing a non-condensable inert gas,
    heating the separation products,
    converting of one or more components of the initial mixture into a gas phase,
    separating one or more components of gas phase and returning the inert gas for interaction with the initial mixture,
    wherein the ratio of the mass of the introduced inert gas to the mass of inert gas providing,
    in aggregate with the vapors of the substance to be removed the same volume of vapor phase at the point of its introduction, as in the process without the introduced inert gas at a lower pressure at the same process temperature, is 0,5-5,0.

2. The method according to claim 1 wherein the introduction and /or withdrawal of the inert gas is carried out in several stages during the separation of the initial mixture.

3. The method according to claim 1 wherein the highly volatile component of the initial mixture is released from the inert gas by cooling.

FIG.1

FIG.2

## INTERNATIONAL SEARCH REPORT

| | International application No. |
|---|---|
| | PCT/RU 2017/000262 |

**A.    CLASSIFICATION OF SUBJECT MATTER**

B01D 3/34 (2006.01); C10G 7/00 (2006.01)

According to International Patent Classification (IPC) or to both national classification and IPC

**B.    FIELDS SEARCHED**

Minimum documentation searched (classification system followed by classification symbols)

B01D 3/00, 3/06, 3/14, 3/16, 3/18, 3/34, 3/38, 3/42, 1/00, 1/14, 1/26, C10G 3/00, 7/00, 7/06

Documentation searched other than minimum documentation to the extent that such documents are included in the fields searched

Electronic data base consulted during the international search (name of data base and, where practicable, search terms used)

PatSearch (RUPTO internal), USPTO, PAJ, K-PION, Esp@cenet, Information Retrieval System of FIPS

**C.    DOCUMENTS CONSIDERED TO BE RELEVANT**

| Category* | Citation of document, with indication, where appropriate, of the relevant passages | Relevant to claim No. |
|---|---|---|
| D, A | SU 1544790 A1 (VSESOIUZNYI NAUCHNO-ISSLEDOVATELSKII INSTITUT PO SBORU, PODGOTOVKE I TRANSPORTU NEFTI I NEFTEPRODUKTOV) 23.02.1990 | 1-3 |
| A | RU 2159792 C1 (PREDPRIIATIE "ASTRAKHANGAZPROM" RAO "GAZPROM") 27.11.2000 | 1-3 |
| A | RU 2170755 C1 (KRIUCHKOV VIKTOR ALEKSEEVICH et al.) 20.07.2001 | 1-3 |
| A | DE 3150137 A1 (LINDE AG) 30.06.1983 | 1-3 |
| A | EA 200200377 A1 (SKAKUNOV IURII PAVLOVICH) 28.08.2003 | 1-3 |
| A | GB 2348826 A (MICHAEL COLE, et al.) 18.10.2000 | 1-3 |

| ☐   Further documents are listed in the continuation of Box C. | ☐   See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 04 September 2017 (04.09.2017) | 07 September 2017 (07.09.2017) |

| Name and mailing address of the ISA/ RU | Authorized officer |
|---|---|
| Facsimile No. | Telephone No. |

Form PCT/ISA/210 (second sheet) (July 1998)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2566775 A, Farrakhov, M. I., Kirichenko S. M., Fahrutdinov R. Z.

- WO 1544790230290 A, Groshev, B., Bronstein, I. S., Kashtanov